# NEW EUROPEAN PATENT SPECIFICATION

(11) **EP 0 601 338 B2**
(45) Date of publication and mention of the opposition decision: **21.09.2005**
(45) Mention of the grant of the patent: 21.10.1998
(21) Application number: 93118002.0
(22) Date of filing: 05.11.1993
(51) Int. Cl.: A61N 1/05, A61N 1/39

(54) **Electrode system for a defibrillator**
Elektrodensystem für ein Defibrillationsgerät
Système d'électrodes pour un defibrillateur

(30) Priority: 11.12.1992 SE 9203732
(43) Date of publication of application: 15.06.1994
(73) Proprietor: St. Jude Medical AB, 175 84 Järfälla (SE)
(72) Inventor: Hirschberg, Jakub, S-183 44 Täby (SE); Bowald, Staffan, S-740 10 Almunge (SE); Wolf, Jens, S-121 45 Johanneshov (SE)
(74) Representative: Harrison, Michael Charles

(56) References cited:
- WO-A-92/11898
- US-A- 4 825 871
- US-A- 5 014 696
- David N. Dunbar et al: "Intracavitary Electrode Catheter Cardioversion of Atrial Tachyarrhythmias in the Dog", JACC Vol.7, No.5, May 1986, pages 1015-1027

## Description

This invention relates generally to an electrode system intended for proximal connection to a cardioverter/defibrillator and for distal placement in the heart region to deliver electrical energy from the cardioverter/defibrillator to the heart so an arrhythmia in the heart can be terminated. More specifically, the invention relates to a system with three electrodes and a defibrillator, said defibrillator being connected to three intravascular electrodes, as stated in the preamble ot claim 1.

In defibrillation/cardioversion ("cardioversion" in this context refers to defibrillation using lower energy; the collective designation "defibrillation" will henceforth be used below) using a three-electrode system with intravascular electrodes, one of the intravascular electrodes is normally placed in the right ventricle and the other either in the superior vena cava or, less commonly, in the inferior vena cava or even in the coronary sinus and its prolongation along the base of the heart. The third electrode is arranged as a subcutaneous electrode with a large area, i.e. a patch electrode, for the purpose of achieving efficient utilization of the energy stored in the heart and good distribution of current in the heart while simultaneously avoiding major surgery (opening of the thorax). The subcutaneous patch electrode is usually placed so in the vicinity of the left ventricle between the patient ts thorax and skin.

Electrode systems with the above-described locations are shown among a plurality of others in US-A-4 708 145. In addition to the intravascular electrodes shown there with a common electrode cable for a plurality of electrodes in the right ventricle and superior vena cava, separate electrode cables for different electrodes, as well as a separate sensing electrode for detecting cardiac events, are also possible. This is shown in US-A-4 727 877, EP-A-O 373 953 and US-A-5 044 375 respectively.

WO 92/11898 discloses a method of inserting an electrode into the coronary sinus by expanding a hollow electrode into contact with the walls of the vein.

A common feature of placements in the prior art systems is an electrode in the right ventricle. However, such an endocardiac electrode, shown in the applicant's previous application, EP-A-559 933, applicant reference GR 92 P 7303, imposes strain on the heart in the form of irritation of the musculature of the heart wall and the valves between the atrium and ventricle. There is a concomitant increased risk of blood clot formation. So the previously cited application proposed an electrode placement in which the endocardiac electrode is avoided and replaced by an electrode in the inferior vena cava.

The purpose of the invention is to achieve an electrode system, without the ventricular electrode, in which efficient utilization of the energy stored in the defibrillator is combined with favorable distribution of current in the heart.

This purpose is achieved with an electrode system, connected to a defibrillator, of the above-described type with the features specified in patent claim 1.

With a design according to the invention, a three electrode system is therefore attained, without a ventricular electrode, for defibrillation with intravascular electrodes, one of which placeable in the coronary sinus including its prolongation along the base of the heart.

Two of the venous electrodes are-arranged along a common electrode cable and have means for fixation to the inner venous wall. The fixation means are in the form of a hollow, resilient cylinder, fixation being achieved through the cylinder's radial expansion. The hollow cylindrical shape of the electrode enables blood to flow unimpeded through the electrode. Other advantageous embodiments are also described in the subclaims

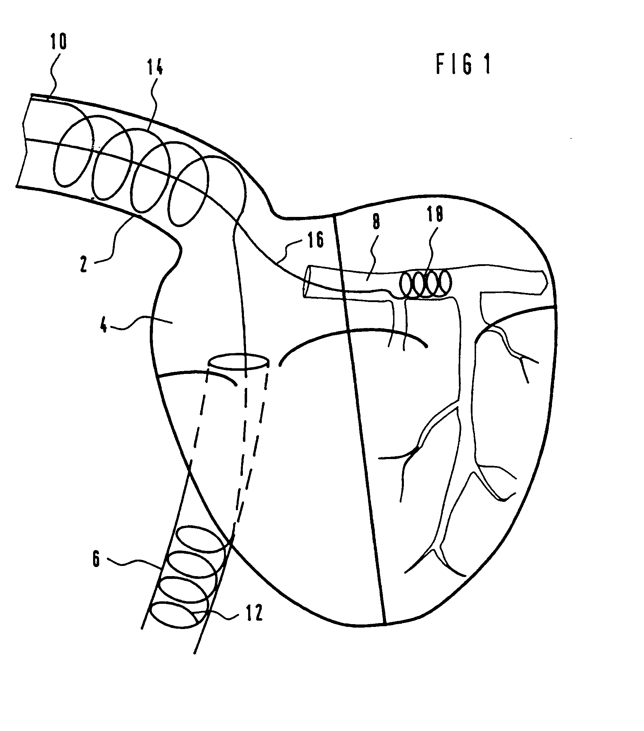

The electrode system according to the invention will be described below, referring to an attached drawing in which
FIG. 1 schematically shows a crossection of a heart (frontalplane) with the electrode system connected to the heart according to an embodiment of the invention.

In FIG. 1, hidden electrode parts in the inferior vena cava and the vein itself are dashed.

FIG. 1 shows a cross-section of a heart and a number of vessels of relevance to the invention. A first electrode cable 10 is introduced through the superior vena cava 2, passes through the right atrium 4 and exits into the inferior vena cava 6. At its distal end, the cable 10 bears an electrode 12 anchored in the inferior vena cava (6). The cable also has an additional electrode 14 anchored in the superior vena cava 2 proximal to the electrode 12. A second electrode cable 16 is also introduced through the superior vena cava 2 but passes through the right atrium 4, in contrast to the cable 10, in such a way that it exits into the coronary sinus 8 and its prolongation, the great cardiac vein, along the base of the heart.

At its distal end, the cable 16 bears an electrode 18 anchored in the coronary sinus/great cardiac vein 8. The electrode 18 is connectable to the defibrillator by a conductor present in the cable 16. The electrodes 12 and 14 are also individually connectable to the defibrillator by separate conductors present in the cable 10.

Connection of the electrodes to the defibrillator and between one another can be achieved in different ways. For example, the electrodes 14, 18 in FIG. 1 can be interconnected so the defibrillation impulse is emitted between these electrodes, on the one hand, and the electrode 12 in the inferior vena cava 6, on the other hand.

However, the electrodes 12, 14, 18 can be interconnected in such a way that the defibrillation pulse is emitted between another one of these electrodes, on the one hand, and the other two electrodes, on the other hand. Alternately, the electrodes 12, 14, 18 can be supplied with different defibrillation voltages or, as an additional alternative, pairs can deliver defibrillation pulses sequentially. Mono-, bi- or multiphasic defibrillation pulses can be used.

Introduction through the inferior vena cava 6 can be an alternative for the described introduction of intravascular electrodes through the superior vena cava 2. The intravascular electrodes have a common electrode cable also in the latter introduction route.

The described electrode configurations can also be complemented with a separate stimulation/sensing electrode for stimulating/sensing cardiac events and/or a sensor for different parameters related to e.g. cardiac hemodynamics. The sensing electrode cable can also contain a stimulation electrode for pacing functions.

The intravascular electrodes 12, 14, 18 are kept in place in the respective vein by being constructed in a way making them radially expandable, then forming at least the contours of a hollow cylinder in the expanded state. Defibrillation electrodes of this type are described in detail in the applicant's simultaneously filed patent application entitled "Defibrillation electrode", EP-A-601 339, this reference forming a part of the present application so as to avoid repetition. The intravascular electrodes 12, 14, 18 are therefore e.g. kept in place in the respective vein by being shaped in the form of helices which apply pressure to the inner wall of the vein by being biased perpendicular to the longitudinal axis of the helix. To avoid repetition, only helical fixation of the electrode 12 in the inferior vena cava 6 will henceforth be described, but the description obviously covers other intravascular electrodes. In its preshaped state, the helical electrode 12 can be envisaged as being coiled around an imaginary cylinder whose external diameter is somewhat larger than the caliber of the inferior vena cava 6. The helix can be made of an electrically conductive, biocompatible material. The electrode 12 is connected to the electrode cable 10 in such a way that they jointly form a single unit. A centrally arranged longitudinal channel through which a stylet can be introduced runs through the electrode 12 and electrode cable 10. In implantation, the electrode 12 is straightened out with the aid of the stylet, the diameter of the electrode 12 thereby becoming smaller than the diameter of the blood vessels it is to traverse and enabling it to be advanced into the inferior vena cava 6. When the implanting physician has decided on an appropriate site for the electrode 12 in the inferior vena cava 6, the stylet can be withdrawn, causing the electrode 12 to resume its preshaped helical configuration. The pressure of the helix against the venous wall keeps the helix in the desired position. In the affixed position, the electrode 12 forms a relatively large electrode surface pressing on the vascular wall. At the same time, the helical electrode 12 has the advantage of enabling blood in the vessel to flow unobstructed through the interior of the helix. The risk of clot formation is thereby minimized. The electrode 12 can also be readily repositioned by reintroduction of the stylet into the central channel to straighten out the electrode 12. The implanting physician can therefore easily find a site for the electrode 12 which, with the other electrodes, achieves a favorable distribution of current in heart tissue.

The implantation can be performed with the aid of a single stylet in a central channel running along the length of the electrodes 12, 14 and the catheter 10. Both electrodes 12 and 14 are then straightened out for implantation. When the electrode 12 has reached the desired position, the stylet is withdrawn enough for the electrode 12 to resume its helical configuration. The electrode 14 is then positioned at the desired site in the superior vena cava 2. When the electrode 14 is in the correct position, the stylet is completely withdrawn so the electrode 14 also resumes its original, preshaped configuration. An introductory catheter can also be used instead of the stylet.

The electrode 18 intended for location in the coronary sinus 8 and its prolongation along the base of the heart can consist of e.g. two sub-electrodes placed in the coronary sinus 8 and its prolongation respectively. The two sub-electrodes can be arranged on a common prolongation of e.g. the electrode cable 16. The two sub-electrodes are placed in the coronary sinus 8 and its prolongation in such a way that an individually adapted, additionally improved distribution of current in the heart is achieved. Here, implantation is performed in the same way as described above. More than two sub-electrodes can be used.

## Claims

1. An electrode system and a defibrillator, said defibrillator being connected to three intravascular electrodes, one (12) of said electrodes being adapted to be placeable in the inferior vena cava (6), an additional intravascular electrode (14) being adapted to be placeable in the superior vena cava (2), the third (18) of said three electrodes (12, 14, 18) being arranged on an electrode cable (16) and being adapted to be placeable in the coronary sinus (8) including its prolongation along the base of the heart, the two electrodes (12, 14) being adapted for placement in the inferior vena cava (6) and the superior vena cava (2) being arranged on a further and common electrode cable (10) at a predetermined distance, the intravascular electrodes (12, 14, 18) in the electrode system having means for affixation to the inner wall of the vein in which they are sited, the fixation means being achieved when the electrodes (12, 14, 18) in the affixed position each has the shape of a hollow, resilient cylinder whose diameter exceeds the diameter of the vein enough for the electrode to press against and affix the electrode to the inner wall of the vein.

2. An electrode system of claim 1, wherein each electrode consists of a helix whose windings form at least part of the cylinder's mantle surface.

3. An electrode system of any of the above claims, wherein the electrode (18) intended for placement in the coronary sinus (8) and its prolongation along the base of the heart consists of at least two sub-electrodes, the distal of the at least two sub-electrodes being connected to the proximal sub-electrode by a prolongation of the electrode cable (16), the sub-electrodes being placeable at separate sites in the coronary sinus (8) including its prolongation along the base of the heart.

## Patentansprüche

1. Ein Elektrodensystem für einen Defibrillator mit drei intavaskulären Elektroden, eine (12) dieser Elektroden angepaßt, in der Vena Cava Inferior (6) plaziert zu werden, eine zusätzliche intravaskuläre Elektrode (14) angepaßt, in der Vena Cava Superior (2) plaziert zu werden, die dritte (18) der drei Elektroden (12, 14, 18) auf einem Elektrodenkabel (16) angeordnet und angepaßt, um in dem Coronari Sinus (8) inklusive seiner Verlängerung entlang der Basis des Herzens plaziert zu werden, die beiden zum Plazieren in der Vena Cava Inferior (6) und der Vena Cava Superior (2) angepaßten Elektroden (12, 14) auf einem weiteren und gemeinsamen Elektrodenkabel (10) in einem vorbestimmten Abstand angeordnet, die intravaskulären Elektroden (12, 14, 18) in dem Elektrodensystem Mittel zum Befestigen an der Innenwand der Vene versehen, in der sie liegen, wobei die Befestigungsmittel erzielt werden, wenn die Elektroden (12, 14, 18) in der befestigten Position jeweils die Form von hohlen, elastischen Zylindern haben, deren Durchmesser den Durchmesser der Vene genügend überschreitet, um die Elektrode gegen die Vene zu drücken un die Elektrode an der Innenwand der Vene zu befestigen.

2. Ein Elektrodensystem nach Anspruch 1, bei dem jede der Elektroden aus einer Wendel besteht, deren Windungen mindestens einen Teil der Mantelfläche des Zylinders bilden.

3. Ein Elektrodensystem nach einem der obigen Ansprüche, bei dem die zur Plazierung in dem Coronari sinus (8) und seiner Verlängerung entlang der Basis des Herzens vorgesehene Elektrode (18) mindestens aus zwei Teilelektroden besteht, wobei die distale der mindestens zwei Teilelektroden mit der porximalen Teilelektrode durch eine Verlängerung des Elektrodenkabels (16) verbunden ist und die Teilelektroden an separaten Stellen in dem Coronari sinus (8) einschließlich seiner Verlängerung entlang der Basis des Herzens plazierbar sind.

## Revendications

1. Système d'électrodes et défibrillateur, le défibrillateur étant connecté à trois électrodes intravasculaires, l'une (12) des électrodes étant conçue pour pouvoir être placée dans la veine cave (6) inférieure, une électrode (14) intravasculaire supplémentaire étant conçue pour pouvoir être placée dans la veine cave (2) supérieure, la troisième (18) des trois électrodes (12, 14, 18) étant disposée sur un câble (16) d'électrode et étant conçue pour pouvoir être placée dans le sinus (8) coronarien en y incluant son prolongement le long de la base du coeur, les deux électrodes (12, 14) adaptées pour être placées dans la veine cave (6) inférieure et la veine cave (2) inférieure étant disposées sur un autre câble (10) d'électrode commun à une distance déterminée à l'avance l'une de l'autre, les électrodes (12, 14, 18) intravasculaires du système d'électrodes ayant des moyens pour la fixation à la paroi intérieure de la veine dans laquelle elles sont situées, les moyens de fixation étant obtenus lorsque les électrodes (12, 14, 18) dans la position de fixation ont chacune la forme d'un cylindre élastique creux dont le diamètre dépasse suffisamment le diamètre de la veine pour que l'électrode s'applique à la paroi intérieure de la veine et se fixe à la paroi intérieure de la veine.

2. Système d'électrodes suivant la revendication 1, dans lequel chaque électrode est constituée d'une hélice dont les enroulements forment au moins une partie de la surface enveloppe du cylindre.

3. Système d'électrodes suivant l'une quelconque des revendications précédentes, dans lequel l'électrode (18) destinée à être mise dans le sinus (8) coronarien et son prolongement le long de la base du coeur est constituée d'au moins deux sous-électrodes, l'électrode distale desdites au moins deux sous-électrodes étant reliée à la sous-électrode proximale par un prolongement du câble (16) d'électrode, les sous-électrodes pouvant être placées en des sites distincts dans le sinus (8) coronarien en y incluant son prolongement le long de la base du coeur.
